# EUROPEAN PATENT APPLICATION

(11) **EP 1 647 777 A2**
(43) Date of publication of application: **19.04.2006**
(21) Application number: 05021836.1
(22) Date of filing: 06.10.2005
(51) Int. Cl.: F24F 3/16

(54) **Apparatus and method for controlling air cleaning in an air conditioner**

(30) Priority: 08.10.2004 KR 2004080447
(71) Applicant: LG ELECTRONICS INC., Seoul 150-010 (KR)
(72) Inventor: Kim, Ho Jung, Inchun-si 403-020 (KR); Choi, In Ho, Kyungki-do 435-040 (KR); Yum, Kwan Ho, Seoul 152-050 (KR); Choi, Ho Seon, Seoul 156-090 (KR)
(74) Representative: Vossius & Partner

(57) **Abstract**

Disclosed is an apparatus and method for controlling an air cleaning. The apparatus includes an air cleaning element (20), a current detector (18), an AC high-voltage generator (15) and a control unit (19) . The air cleaning element (20) generates negative ions and ozone using a plasma discharge and cleans indoor air by the negative ions and ozone. The current detector (18) detects a state of current passing through the air cleaning element (20). The AC high-voltage generator (15) generates an AC high-voltage, and applies the AC high-voltage to the air cleaning element (20). The control unit (19) varies a magnitude of the AC voltage applied to the air cleaning element(20) by the AC high-voltage generator (15) depending on a current detecting signal received from the current detector(18). Therefore, ozone is adequately generated below a limit value depending on a state of indoor air, thereby increasing air cleaning performance so as to sterilize and clean contaminants more efficiently.

## Description

The present invention relates to an air cleaning apparatus arranged within an air conditioner, an air cleaner, or the like, and more particularly to an apparatus and method for controlling air cleaning, which is capable of cleaning indoor air by detecting current passing through an air cleaning element and adequately controlling the amount of generation of ozone depending on the detected current.

An air cleaning apparatus generates a number of negative ions using an air cleaning element, and thereby cleans indoor air using such generated negative ions, wherein the air cleaning element is referred to as a negative ion generator of a surface discharge type which has recently been developed.

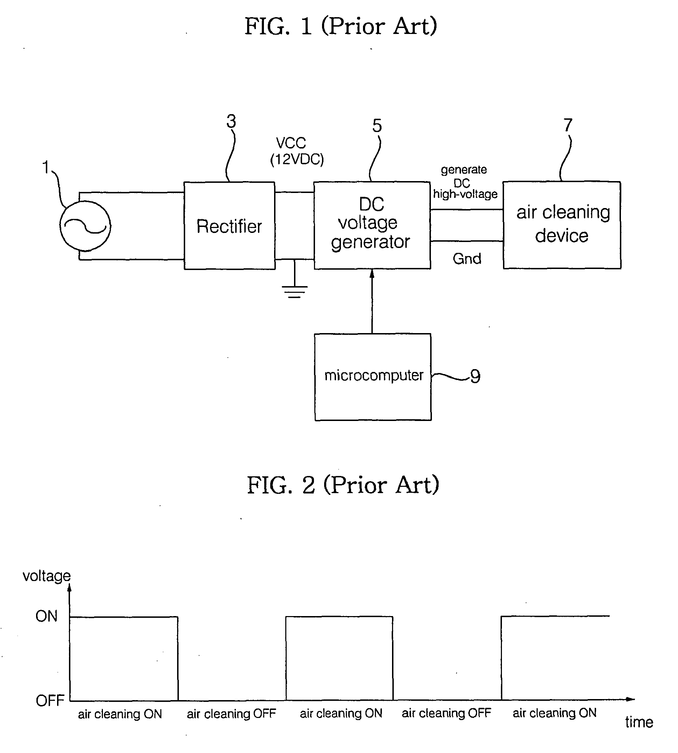

FIG. 1 is a block diagram showing the construction of a conventional air cleaning control apparatus, and FIG. 2 is a graph illustrating an operating control state of the conventional air cleaning control apparatus. Referring to FIGS. 1 and 2, the conventional air cleaning control apparatus using the above air cleaning element will be explained as follows.

The conventional air cleaning control apparatus includes, as shown in FIG. 1, a rectifier 3, a DC voltage generator 5, an air cleaning element 7 and a microcomputer 9. The rectifier 3 rectifies an AC voltage into a DC voltage, for example, it may rectify a commercial AC voltage of 220V into a 12V DC voltage. When the 12V DC voltage is supplied to the DC voltage generator 5, the DC voltage generator 5 supplies a high voltage to the air cleaning element 7 so as to generate negative ions.

As shown in FIG. 2, the microcomputer 9 generates ON/OFF signals in response to an operation signal and a stop signal manually inputted by a user, wherein the ON signal is converted into a high-voltage signal by the DC voltage generator 5, and the OFF signal is converted into a ground voltage signal by the DC voltage generator 5. Thus, any one of the high-voltage ON signal and the ground OFF signal is applied to the air cleaning element 7.

For example, when the microcomputer 9 supplies the ON signal to the DC voltage generator 5, the high-voltage ON signal is generated by the DC voltage generator 5. In addition, the high-voltage ON signal is applied to the air cleaning element 7, thereby generating negative ions by means of plasma discharge in the air cleaning element 7.

The negative ions generated in the air cleaning element 7 are supplied to indoor air passing through the vicinity of the air cleaning element 7, thereby cleaning indoor air.

However, the above conventional air cleaning control apparatus has a structure for merely generating the negative ions and cleaning indoor air, without regard to a state of indoor air. Even when indoor air is relatively seriously polluted by some contaminants such as cigarette smoke, dust, etc., the air cleaning element 7 operates uniformly without regard to a pollution state. Thus, because the air cleaning element 7 does not operate in correspondence with the pollution level of indoor air, it does not effectively clean indoor air.

Particularly, the above conventional air cleaning control apparatus has the DC high-voltage generator 5, but it is not possible to easily control characteristics of the DC high-voltage generator 5. For this reason, even though the DC high-voltage generator 5 is used, it is difficult to easily generate ozone below a predetermined limit value using the air cleaning element 7. Consequently, even though ozone has a high sterilization effect, because ozone is not easily generated below the predetermined limit value, it is difficult to sterilize and clean indoor air.

Therefore, the present invention has been made in view of the above problems, and it is an object of the present invention to provide an apparatus and method for controlling air cleaning, wherein, when ozone generation is controlled by controlling an AC voltage applied to an air cleaning element and detecting current passing through the air cleaning element, ozone below a limit value is adequately generated depending on a state of indoor air, thereby increasing an air cleaning performance so as to sterilize and clean contaminants more efficiently.

In accordance with an aspect of the present invention, the above and other objects can be accomplished by the provision of an apparatus for controlling air cleaning, the apparatus comprising: an air cleaning element 20 for generating negative ions and ozone using plasma discharge, and cleaning air using the negative ions and ozone; a current detector 18 for detecting a state of current passing through the air cleaning element 20; an AC high-voltage generator 15 for generating an AC high voltage, and supplying the AC high-voltage to the air cleaning element 20; and a control unit 19 for varying a magnitude of the AC voltage applied to the air cleaning element 20 by the AC high-voltage generator 15, depending on a current detecting signal received from the current detector 18.

Preferably, the apparatus further comprises a rectifier 13 for rectifying an AC voltage into a DC voltage with a fixed voltage and supplying the rectified DC voltage to the AC high-voltage generator 15.

Preferably, the current detector 18 is arranged on a ground circuit 16 which is connected to the air cleaning element 20.

Preferably, the apparatus further comprises a pollution level measuring instrument 17 for measuring the pollution level of indoor air and supplying a pollution level signal to the control unit 19, wherein the control unit 19 varies the magnitude of the AC voltage applied to the air cleaning element 20 by the AC high-voltage generator 15.

In accordance with another aspect of the present invention, there is provided a method for controlling air cleaning, the method comprising the steps of: a) detecting a magnitude of current passing through the air cleaning element; and b) lowering a magnitude of an AC voltage applied to an air cleaning element by an AC high-voltage generator, or turning a power OFF, when the magnitude of the detected current is over a predetermined value.

Preferably, the step b) is performed during a predetermined period of time.

Preferably, the magnitude of the AC voltage is lowered to a control level of "medium" or "small" when the AC voltage applied to the air cleaning element depending on the pollution level is set to a control level of "large", and the magnitude of the AC voltage is lowered to the control level of "small" when the AC voltage is set to the control level of "medium", thereby decreasing ozone generation in the air cleaning element.

In a feature of the present invention, when the AC voltage applied to the air cleaning element depending on the pollution level of indoor air is adequately controlled, and current passing through the air cleaning element is detected, it is possible to adequately generate ozone below a limit value depending on a state of indoor air, thereby enhancing air cleaning performance so as to sterilize and clean contaminants included in the air more efficiently.

### BRIEF DESCRIPTION OF THE DRAWINGS

The above and other objects, features and other advantages of the present invention will be more clearly understood from the following detailed description taken in conjunction with the accompanying drawings, in which:
FIG. 1 is a block diagram showing the construction of a conventional air cleaning control apparatus;
FIG. 2 is a graph illustrating an operating control state of the conventional air cleaning control apparatus;
FIG. 3 is a block diagram showing the construction of an air cleaning control apparatus according to the present invention;
FIG. 4 is a perspective view illustrating an air cleaning element used in the present invention;
FIG. 5 is a graph illustrating AC voltage control signals in an air cleaning control apparatus according to the present invention;
FIG. 6 is a graph illustrating control states of current detection in an air cleaning control apparatus according to the present invention; and
FIG. 7 is a flowchart illustrating a control method using an air cleaning control apparatus according to the present invention.

### DESCRIPTION OF THE PREFERRED EMBODIMENTS

Now, preferred embodiments of the present invention will be described in detail with reference to the annexed drawings.

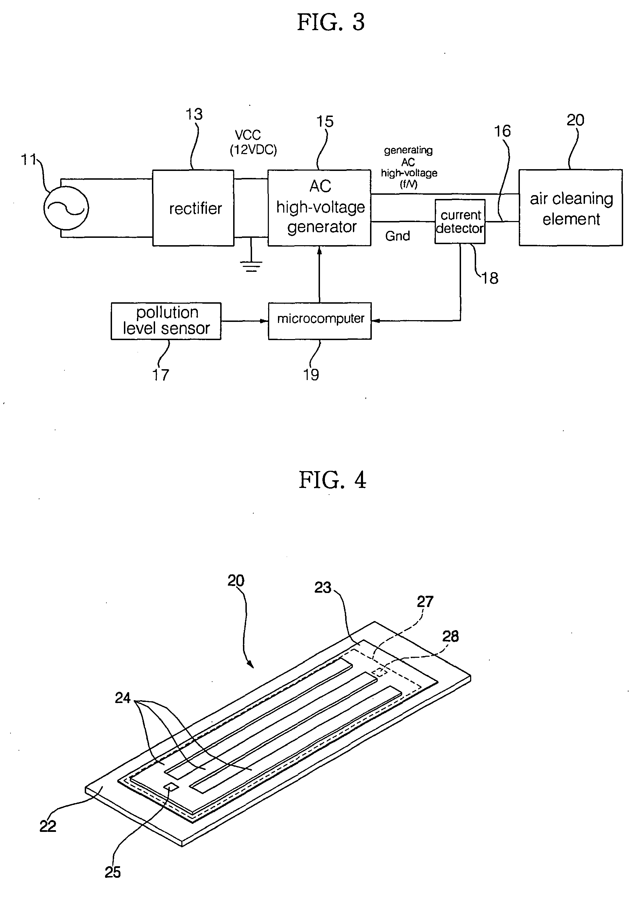

FIG. 3 is a block diagram showing the construction of an air cleaning control apparatus according to the present invention, and FIG. 4 is a perspective view illustrating an air cleaning element used in the present invention.

The air cleaning control apparatus according to the present invention includes, as shown in FIG. 3, an air cleaning element 20 for generating negative ions and ozone using plasma discharge, and for sterilizing and cleaning indoor air, a current detector 18 for detecting current passing through the air cleaning element 20, an AC high-voltage generator 15 for applying an AC voltage to the air cleaning element 20, and a control unit such as a microcomputer 19 for varying the AC voltage applied to the air cleaning element 20 by the AC high-voltage generator 15 depending on a current detecting signal received from the current detector 18.

The air cleaning element 20 includes, as shown in FIG. 4, a discharge electrode 24 and a ground electrode 27. The discharge electrode 24 is formed on an upper surface of an insulating dielectric substrate 22 with a substantially rectangular shape. The ground electrode 27 is formed on a bottom surface opposite the upper surface. The discharge electrode 24 and the ground electrode 27 are protected by a protective film coated on both surface sides of the insulating dielectric substrate 22.

The discharge electrode 24 is formed to expose a supply terminal 25 for supplying an input voltage of the AC high-voltage generator 15. The ground electrode 27 is formed to expose a ground terminal 28 which is connected with a ground circuit 16.

The current detector 18 is a device for detecting current passing through the ground terminal 28 when the AC voltage applied to the air cleaning element 20 generates plasma discharge. It is preferred that the current detector 18 be arranged on the ground circuit 16 which is connected with the AC high-voltage generator 15, etc., at the ground terminal 28.

The AC high-voltage generator 15 is formed, as shown in FIG. 3, to supply a voltage by way of the rectifier 13, wherein the rectifier 13 rectifies the AC voltage such as a commercial voltage, etc., into a DC voltage, for example, a 12V DC voltage. ,

The AC high-voltage generator 15 applies the AC voltage to the air cleaning element 20, wherein the AC voltage is easily controlled due to characteristics thereof. When a high-voltage over a predetermined voltage is applied to the air cleaning element 20, it is possible to generate ozone as well as negative ions, the ozone having an excellent capability for sterilizing contaminants. In addition, when the AC voltage applied to the air cleaning element 20 is varied, it is possible to adequately control the generation of ozone as well.

The microcomputer 19 is formed to receive a pollution measuring signal transmitted from a pollution level sensor which measures a state of indoor air pollution.

In the case where the pollution level of indoor air is measured by the pollution level sensor 17, when the pollution level of indoor air is high the microcomputer 19 raises the AC voltage applied to the air cleaning element 20. While the pollution level of indoor air is low, the microcomputer 19 lowers the AC voltage applied to the air cleaning element 20.

Consequently, the above microcomputer 19 controls the above AC high-voltage generator 15 so that the air cleaning element 20 generates ozone and negative ions. The concrete control procedures will be explained as follows.

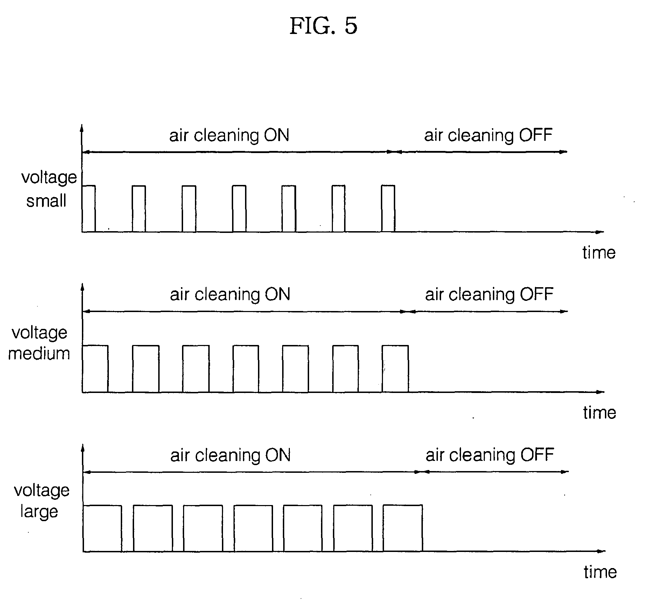

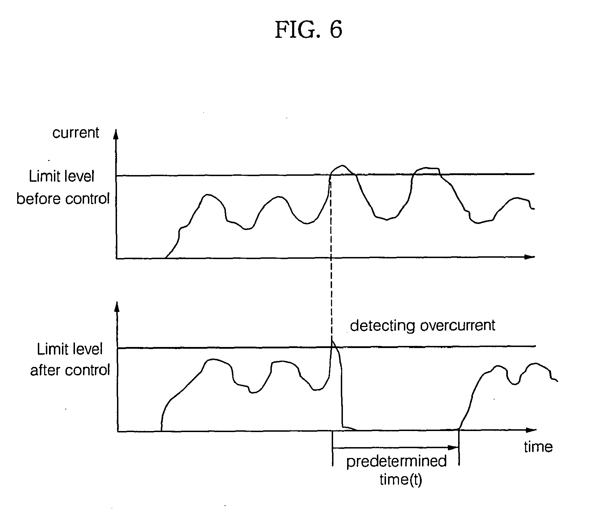

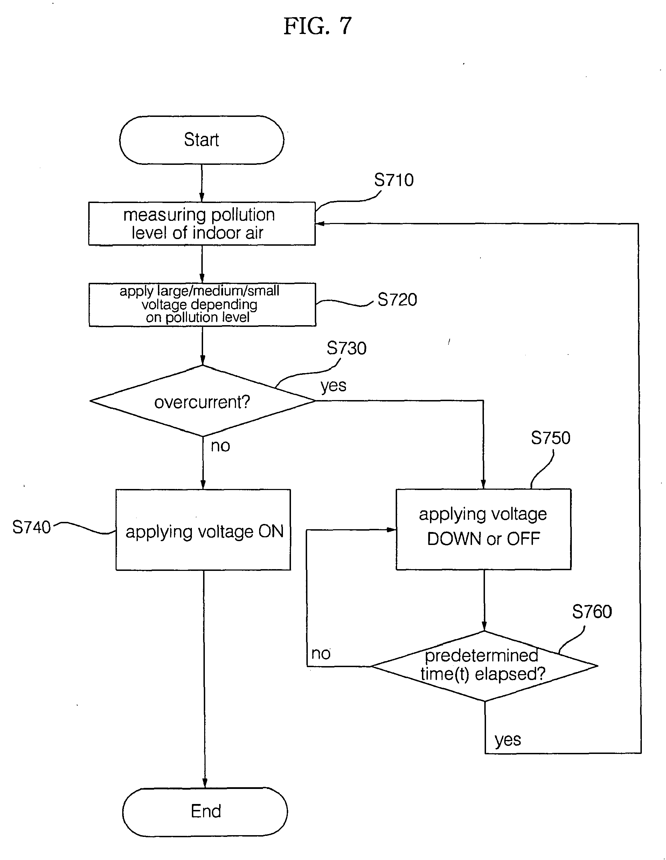

FIG. 5 is a graph illustrating AC voltage control signals in an air cleaning control apparatus according to the present invention, FIG. 6 is a graph illustrating control states of current detection in an air cleaning control apparatus according to the present invention, and FIG. 7 is a flowchart illustrating a control method using an air cleaning control apparatus according to the present invention.

To begin with, referring to FIG. 3, when the air cleaning control apparatus operates, a 220V AC voltage as a commercial voltage 11 is rectified into a 12V DC voltage by the rectifier 13. Then, the DC voltage is supplied to an AC high-voltage generator 15.

Referring to FIG. 7, the pollution level sensor 17 measures the pollution level of indoor air (S710), and transmits a pollution level signal. The microcomputer 19 receives the pollution level signal from the pollution level sensor 17, and determines the pollution level of indoor air.

As shown in FIG. 5, when the microcomputer 19 determines that the pollution level of indoor air is over a predetermined level, it controls the AC high-voltage generator 15, raising the AC voltage applied to the air cleaning element 20 by the AC high-voltage generator 15.

Meanwhile, when the microcomputer 19 determines that the pollution level of indoor air is below the predetermined level, it controls the AC high-voltage generator 15, lowering the AC voltage applied to the air cleaning element 20 by the AC high-voltage generator 15.

That is, when the microcomputer 19 determines the pollution level of indoor air, as shown in FIG. 5, the magnitude of the AC voltage applied to the air cleaning element 20 is set to a control level of "large", "medium" or "small" (S720), the AC voltage being controlled by a Pulse Width Modulation (PWM) method.

When the AC high-voltage generated by the AC high-voltage generator 15 depending on the above pollution level is applied to the air cleaning element 20, the air cleaning element 20 generates ozone and negative ions by a plasma discharge phenomenon depending on the control level. Thus, the ozone and negative ions can sterilize and clean harmful gases included in indoor air.

Meanwhile, when the air cleaning element 20 operates, AC current applied to the air cleaning element 20 induces plasma discharge and flows into the ground circuit 16 through the ground terminal 28. Since the amount of current flowing into the ground circuit 16 generally is in proportion to the amount of ozone generated in the air cleaning element 20.

Therefore, the microcomputer 19 determines whether current detected by the current detector 18 arrives, as shown in FIG. 6, at overcurrent over a predetermined level (S730). When current detected by the current detector 18 does not arrive at overcurrent, the AC voltage is applied to the air cleaning element 20 (S740). While current detected by the current detector 18 arrives at overcurrent, it is determined that a density of ozone generated at the surface of the air cleaning element 20 arrives at a limit value over a predetermined level, thereby turning off the AC voltage applied to the air cleaning element 20 during a predetermined period of time in order to suppress the generation of ozone (S750, S760).

On the other hand, when the AC voltage applied to the air cleaning element 20 depending on the pollution level is set to a control level of "large", the magnitude of the AC voltage is lowered to a control level of "medium" or "small", and when the AC voltage is set to the control level of "medium", the magnitude of the AC voltage is lowered to the control level of "small" (S750). Thus, it is possible to decrease the amount of ozone generated in the air cleaning element 20.

When the AC high-voltage applied to the air cleaning element 20 is turned ON/OFF, depending on the pollution level and the magnitude of current passing through the air cleaning element 20, or when a magnitude of the applied AC voltage is varied, the amount of the plasma discharge and the amount of ozone generated in the air cleaning element 20 are varied, respectively. Therefore, by adequately generating the negative ions as well as ozone below a limit value, it is possible to sterilize and clean indoor air more efficiently.

As apparent from the above description, the present invention provides an apparatus and method for controlling air cleaning, wherein, when a generation of the amount of ozone is controlled by controlling an AC voltage applied to an air cleaning element and detecting current passing through the air cleaning element, ozone is adequately generated below a limit value depending on a state of indoor air, thereby increasing air cleaning performance so as to sterilize and clean contaminants more efficiently.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. An apparatus for controlling air cleaning, the apparatus comprising:
an air cleaning element (20) for generating negative ions and ozone using plasma discharge, and cleaning air using the negative ions and ozone;
a current detector (18) for detecting a state of current passing through the air cleaning element (20);
an AC high-voltage generator (15) for generating an AC high voltage, and supplying the AC high-voltage to the air cleaning element (20); and
a control unit (19) for varying a magnitude of the AC voltage applied to the air cleaning element (20) by the AC high-voltage generator (15), depending on a current detecting signal received from the current detector(18).

2. The apparatus as set forth in claim 1, further comprising a rectifier(13) for rectifying an AC voltage into a DC voltage with a fixed voltage and supplying the rectified DC voltage to the AC high-voltage generator(15).

3. The apparatus as set forth in claim 1 or 2, wherein the current detector(18) is arranged on a ground circuit(16) which is connected with the air cleaning element(20).

4. The apparatus as set forth in any of claims to 3, further comprising a pollution level measuring instrument (17) for measuring the pollution level of indoor air and supplying a pollution level signal to the control unit (19), wherein the control unit (19) varies the magnitude of the AC voltage applied to the air cleaning element(20) by the AC high-voltage generator (15).

5. A method for controlling air cleaning, the method comprising the steps of:
a) detecting a magnitude of current passing through an air cleaning element; and
b) lowering a magnitude of an AC voltage applied to the air cleaning element by an AC high-voltage generator, or turning a power OFF, when the magnitude of the detected current is over a predetermined value.

6. The method as set forth in claim 5, wherein the step b) is performed during a predetermined period of time.

7. The method as set forth in claim 6 or 7, wherein the magnitude of the AC voltage is lowered to a control level of "medium" or "small" when the AC voltage applied to the air cleaning element depending on the pollution level is set to a control level of "large", and the magnitude of the AC voltage is lowered to the control level of "small" when the AC voltage is set to the control level of "medium", thereby decreasing ozone generation in the air cleaning element.
